# EUROPEAN PATENT SPECIFICATION

(11) **EP 1 797 845 B1**
(45) Date of publication and mention of the grant of the patent: **19.10.2016**
(21) Application number: 06256415.8
(22) Date of filing: 18.12.2006
(51) Int. Cl.: A61F 2/08

(54) **Arthroscopic implants with integral fixation devices**
Arthroskopie-Implantat mit eingebauten Befestigungsvorrichtungen
Implant arthroscopique avec éléments de fixation intégrés

(30) Priority: 19.12.2005 US 311792
(43) Date of publication of application: 20.06.2007
(73) Proprietor: DePuy Mitek, LLC, Raynham, MA 02767 (US)
(72) Inventor: Whittaker, Gregory R., Stoneham, MA 02180 (US)
(74) Representative: Tunstall, Christopher Stephen

(56) References cited:
- EP-A2- 1 199 036
- WO-A-99/21515
- FR-A1- 2 683 715
- US-A- 5 067 962
- US-A1- 5 004 474
- US-A1- 5 871 504
- US-A1- 2004 102 780
- US-A1- 2006 271 192

## Description

### FIELD OF THE INVENTION

This invention relates to implants for arthroscopic surgical procedures, in particular to implants for replacing an anterior cruciate ligament in the knee.

### BACKGROUND OF THE INVENTION

Arthroscopic surgical repairs of a ruptured anterior cruciate ligament in the knee are known in this art. A rupture of the anterior cruciate ligament ("ACL") is often seen in sports related injuries. In a conventional arthroscopic ACL reconstruction procedure, the surgeon prepares the patient for surgery by insufflating the patient's knee with sterile saline solution. Several cannulas are inserted into the knee and used as entry portals into the interior of the knee. A conventional arthroscope is inserted through one of the cannulas so that the surgeon may view the surgical site remotely. The surgeon then drills a tibial tunnel and a femoral tunnel in accordance with conventional surgical techniques using conventional surgical drills and drill guides. A replacement anterior cruciate ligament graft is then prepared and mounted in the tibial and femoral tunnels, and secured using conventional techniques and known fixation devices in order to complete the knee reconstruction.

Several types of anterior cruciate ligament grafts are available for use by the surgeon in ACL reconstruction. The grafts may be autografts that are harvested from the patient, for example, patellar bone-tendon-bone grafts, or hamstring grafts. Alternatively, the grafts can be xenografts, allografts, or may be prepared using natural or synthetic polymers. There are various known methods of securing these ACL grafts in bone tunnels. These methods include the use of fixation devices such as one or more cross-pin intersecting the tunnel to retain the graft, interference screws driven between the graft and a wall of the bone tunnel, or any of various other retention devices applied during surgery for positioning, tensioning and securing the graft.

Although the existing methods for performing ACL reconstruction are satisfactory for their intended purpose, and generally provide the patient with the desired therapeutic result, these surgical procedures are considered by some to be complex and generally leave one or more implants, such as cross pins or interference screws, in the patient. In certain cases it is believed that implants may trigger immune responses in the patient, or otherwise interfere with healing, for example, by reducing the area of direct contact between the ACL graft tendon and the patient's native tissue. It would thus be desirable to reduce or eliminated the use of fixation devices in ACL surgery, and particularly the use of fixation devices remaining in the patient after surgery.

Accordingly, there is a need in this art for improved devices and methods of ACL reconstruction having reduced complexity and reduced dependence on fixation devices that behave post-surgically as foreign bodies in the patient.

FR-A-2683715 discloses an implant in accordance with the preamble of claim 1.

### SUMMARY OF THE INVENTION

The present invention relates to arthroscopic implants, and particularly to implants for replacing an anterior cruciate ligament in the knee. It is an object of the present invention to provide a surgical implant that includes one or more integrated fixation device for deployment in a bone tunnel during arthroscopic surgery such as ACL repair surgery, thereby reducing or eliminating the requirement for additional fixation devices to position and retain the implant.

It is a further object of the present invention to provide an integrated implant for arthroscopic surgery that can be implanted and tensioned using a single installation tool.

Accordingly, an implant is disclosed for replacing an anterior cruciate ligament in a knee. The implant includes a first fixation member having an axis and a first substantially cylindrical external surface about the axis. The first surface defines first external screw threads adapted for threaded engagement with first internal screw threads formed in a tunnel in a tibia adjacent to the knee. The first external screw threads have a first major thread diameter and a first minor thread diameter. The implant also includes a second fixation member having a second substantially cylindrical external surface about the axis. The second surface defines second external screw threads adapted for threaded engagement with second internal screw threads formed in a tunnel in a femur adjacent to the knee. The second external screw threads have a second major thread diameter and a second minor thread diameter. The implant also includes a flexible graft ligament member interconnecting the first fixation member and the second fixation member. The second major thread diameter is smaller than the first minor thread diameter, so that the second fixation member can be passed through the tunnel in the tibia without engaging the internal screw threads therein.

In one embodiment, the first fixation member, the second fixation member and the ligament member are made using allograft tissue. In another embodiment, the first fixation member, the second fixation member and the ligament member are made using xenograft tissue. In a further embodiment, at least one of the first and the second fixation member is reinforced with a biocompatible material. In another embodiment, the first and the second fixation member and the ligament member are manufactured using synthetic biocompatible material.

The implant may also include an axial bore through the first fixation member and at least partially through the second fixation member. In different embodiments, the axial bore has a polygonal or an oval internal cross section. The axial bore is adapted for sliding engagement with an insertion tool for the implant, the tool including a proximal handle and a distal shaft fixed to the handle and adapted for sliding engagement with the axial bore. When the implant is mounted on the tool, the handle is positioned outside the axial bore. Either or both of the first and the second fixation member may also include one or more transverse bore in fluid communication with the axial bore and with the respective external surface. The one or more transverse bore can be used to deliver a fluid to a surgical site. For delivering the fluid to the surgical site, the tool may include a cannulation through the handle and at least partially through the shaft.

Another aspect of the present invention is a kit including the tool and the implant, as described above. The implant in the kit may also include the one or more transverse bore and the cannulated tool.

Also disclosed herein is a method for replacing an anterior cruciate ligament in a knee. The method includes steps of forming an internally screw threaded first tunnel through a tibia adjacent to the knee, and forming an internally screw threaded second tunnel in a femur adjacent to the knee. The method also includes providing a threaded implant as described herein, the implant having an axial bore for receiving an insertion tool, and providing the insertion tool including a handle fixed to an elongated shaft adapted for sliding engagement with the axial bore through the first fixation member and at least partially through the second fixation member.

The method further includes slidingly engaging the tool with the first and the second fixation member, passing the second fixation member through the internally threaded first tunnel, and simultaneously rotationally threading the first fixation member into the internally threaded first tunnel and the second fixation member into the internally threaded second tunnel. The second fixation member is passed axially through the first tunnel without screw thread engagement.

The method may also include steps of disengaging the tool from the second fixation member while leaving the tool engaged with the first fixation member, tensioning the implant by rotating the tool and the first fixation member engaged therewith, then disengaging the tool from the first fixation member. The method may include injecting a fluid through the implant, wherein the implant defines at least one transverse bore in fluid communication with the axial bore and at least one of the first and the second external surface, the tool further including a cannulation in fluid communication with the at least at least one transverse bore. In various embodiments, the fluid is an adhesive, a medicant, or a lubricant.

These and other aspects of the present invention will be more apparent from the following description and accompanying drawings.

### BRIEF DESCRIPTION OF THE DRAWINGS

FIG. 1 illustrates an implant not in accordance with the present invention including a ligament graft with an integral externally threaded fixation members at proximal and distal ends, the fixation members being of substantially equal diameter; the implant is shown in a knee..
FIG. 2 illustrates a proximal end view of the implant of FIG. 1, the implant including a polygonal cross section longitudinal bore for receiving an insertion tool.
FIG. 3 illustrates an end view of another implant of FIG. 1, the implant including an oval cross section longitudinal bore for receiving an insertion tool.
FIG. 4 illustrates an implant not in accordance with the present invention including a ligament graft with fully circumferentially threaded fixation members at proximal and distal ends.
FIG. 5 illustrates an embodiment of an implant of the present invention including a ligament graft with externally threaded fixation members at proximal and distal end ends, the fixation members being of unequal diameter.
FIG. 6 illustrates an embodiment of an implant of the present invention including a ligament graft having externally threaded fixation members at proximal and distal ends, at least one of the fixation members including fluid injection ports.
FIG. 7 illustrates an embodiment of a polygonal external cross section insertion tool for a threaded implant of the present invention having a mating, polygonal internal cross section longitudinal bore.
FIG. 8 illustrates an embodiment of a cannulated insertion tool for an implant of the present invention, for delivering a fluid to a surgical site.
FIG. 9 illustrates an embodiment of an oval external cross section insertion tool for an implant of the present invention having a mating oval internal cross section longitudinal bore.
FIGs. 10 through 13 illustrate an embodiment of a procedure for mounting a threaded implant of the present invention in a joint.

### DETAILED DESCRIPTION OF THE INVENTION

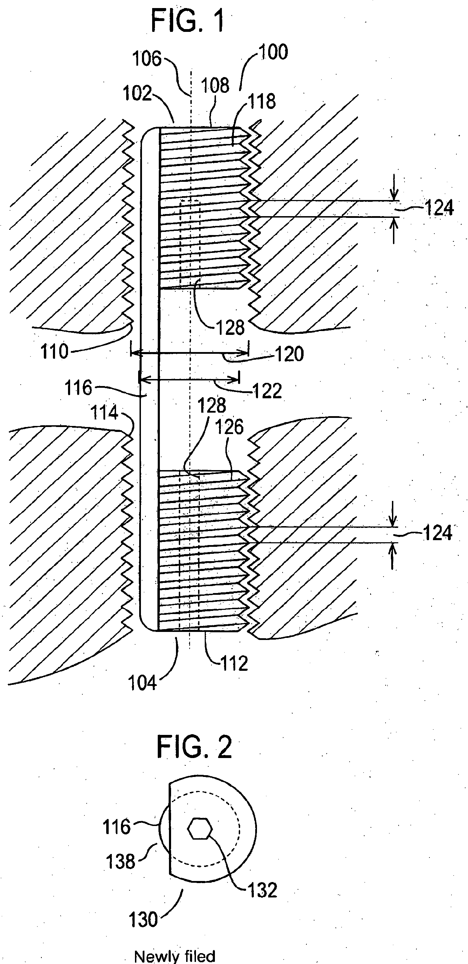

Referring more particularly to the figures, FIG. 1 illustrates a threaded implant 100. The implant 100 has a distal end 102, a proximal end 104, and a longitudinal axis 106 extending therebetween. The implant 100 also includes a distal fixation member 108 for fixing the implant into a first internally threaded tunnel in bone 110, a proximal fixation member 112 for fixing the implant into a second internally threaded tunnel in bone 114 and an interconnecting flexible ligament member 116 extending between the distal fixation member 108 and the proximal fixation member 112. The implant 100 can be fashioned from harvested allograft or xenograft tissue suitable for a bone-tendon-bone implant, or manufactured from natural or synthetic materials including biocompatible polymers, ceramics, minerals, and combinations thereof. The flexible ligament member 116 may be integral with one or both of the distal 108 and the proximal fixation member 112, or the distal fixation member 108, the proximal fixation member 112 and the flexible ligament member 116 may be separately harvested or manufactured components that are assembled to form the implant 100. In an embodiment, the implant 100 is a BTB allograft, and each of the distal 108 and the proximal fixation member 112 is formed from a harvested bone block at an end of the allograft.

The distal fixation member 108 is substantially cylindrical in shape and includes external screw threads 118 centered about the longitudinal axis 106 and having a major thread diameter 120, a minor thread diameter 122, and a longitudinal thread spacing 124. By major thread diameter of an external screw thread, we mean the outer diameter of an external screw thread measured at peaks of the threads. A major thread diameter of a threaded fixation member corresponds to an outer diameter of the fixation member. By minor thread diameter of an external screw thread, we mean the diameter of an external screw thread measured from the bottom of valleys between the threads. Longitudinal thread spacing (thread spacing) is the longitudinal distance between adjacent threads, and is inversely related to thread pitch, that is, thread pitch equals 1/thread spacing. Thread depth is the radial distance between the peaks and valleys of the thread, equal to half of the difference between the major thread diameter and the minor thread diameter.

The proximal fixation member 112 is substantially cylindrical in shape has external screw threads 126 of substantially the same screw thread specifications with regard to major diameter 120, minor diameter 122 and thread spacing 124 as the distal fixation member 108. Screw threads may be formed on the implant 100 by any thread forming method suitable for fabricating a surgical implant. Examples of suitable external thread-forming methods for the distal 108 and proximal fixation member 112 may include mechanical thread cutting, laser or water-jet cutting, and pressure forming. Preparation of the implant may also include reinforcing one or both bone blocks with a biocompatible material such as a bone cement, an apatite composition, or a curable polymeric material. The reinforcing material may be a bioreplaceable material or a nonabsorbable material or a combination thereof.

Fixation members of the present invention are sized similarly to bone blocks used in conventional ACL repair surgery, typically in the range of 15 millimeters to forty millimeters in length, and eight millimeters to twenty millimeters in diameter. In one embodiment of the present invention, a major thread diameter of a fixation member is in the range of eight to twenty millimeters. In another embodiment, the major thread diameter is in the range of twelve to fifteen millimeters. In an embodiment, the thread pitch is in the range of six to twelve threads per 2.54 cm (inch) and the thread depth is in the range of 1 mm to 3.2 mm (0.04 inches to 0.125 inches).

The implant 100 also includes an axial bore 128 through the proximal fixation member 112 and at least partially through the distal fixation member 108. In the implant illustrated in FIG. 1, the axial bore 128 in the distal fixation member 108 is closed at the distal end 102. In another implant, the distal fixation member 108 is bored through to the distal end 102. The axial bore 128 is adapted for internal sliding, removable engagement with a tool for rotating the implant 100 about the axis 106, for threading the implant 100 through the second internally threaded tunnel 114 and into the first internally threaded tunnel 110. In one variant, the implant 100 is an ACL graft implant, the second internally threaded tunnel 114 is a bore in a tibia and the first internally threaded tunnel 110 is in a femur. In another variant, fixation of the implant 100 in a threaded bone tunnel requires as fixation means only the external threads 118, 126 fashioned from the material of respective bone blocks or their synthetic equivalents. That is, the fixation means for the implant 100 is unitary with the implant 100.

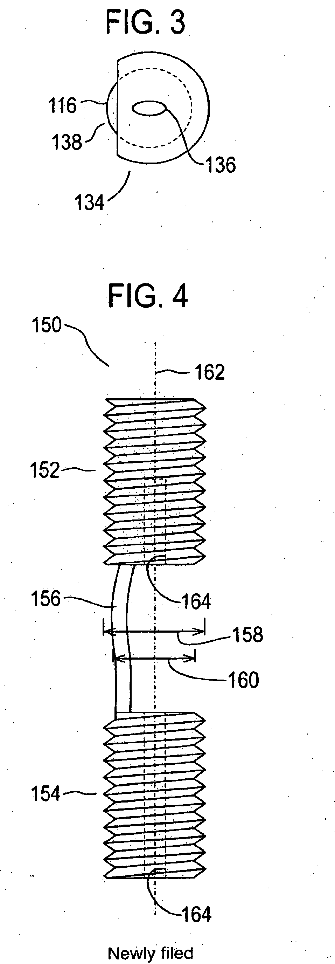

FIG. 2 illustrates a proximal end view 130 of the implant 100 of FIG. 1, wherein the axial bore 128 has a hexagonal cross section 132. The cross section of the bore 128 may also be another cross section, such as another polygonal cross section, adapted for sliding, removable engagement with an insertion tool having a corresponding external cross section. FIG. 3 illustrates a proximal end view 134 of another variant of the implant 100 of FIG. 1, wherein the bore 128 has an oval cross section 136 for sliding, removable engagement with an insertion tool having a corresponding oval external cross section. By an oval cross section, we mean any elongated cross section having rounded ends, for example, an ellipse or a flat-sided elongated shape with rounded ends.

The external screw threads 118, 126 of the implant 100 are interrupted at a circumferential position 138 about the axis 106, corresponding to the orientation of the flexible ligament member 116 of the implant. The interruption of the threads 118, 126 is included to avoid damage to the ligament member 116 of the implant 100 when the threads 118, 126 are formed or when the implant 100 is threaded into the first 110 or the second internally threaded tunnel. FIG. 4 illustrates another implant 150 that does not require circumferential interruption of external screw threads. The implant 150 includes a threaded distal fixation member 152, a threaded proximal fixation member 154, and a flexible ligament member 156 interconnecting the distal 152 and the proximal fixation member 154. Each of the distal 152 and proximal fixation member 154 has a major screw thread diameter 158 and a minor screw thread diameter 160. The flexible ligament member 152 is seen to be positioned completely within the minor thread diameter 160 of both the distal 152 and the proximal fixation member154, thereby preventing damage to the flexible ligament member 156 when the implant 150 is threaded into mating internally threaded bone tunnels. The implant 150 is also seen to have a longitudinal axis 162 and an axial bore 164 through the proximal fixation member 154 and into the distal fixation member 152, for sliding, removable engagement with an insertion tool.

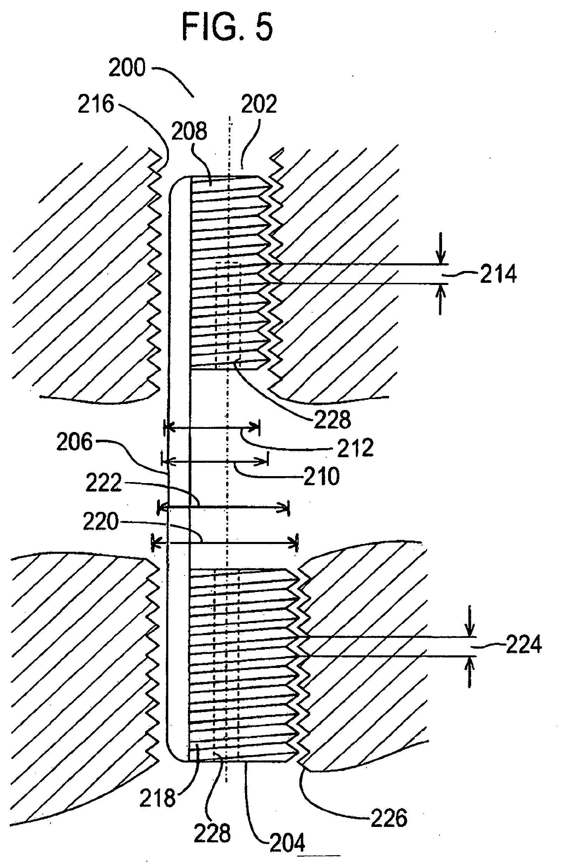

FIG. 5 illustrates an embodiment of a dual-diameter threaded implant 200 according to the present invention. The dual diameter implant 200 is constructed in a similar manner to the threaded implant 100 illustrated in FIG. 1, but the dual diameter implant 200 includes an externally threaded distal fixation member 202 that is smaller in diameter than an externally threaded proximal fixation member 204. The dual diameter implant also includes an interconnecting flexible ligament member 206 extending between the distal 202 and the proximal fixation member 204. The proximal fixation member 204 has first screw threads 218 having a first major thread diameter 220, a first minor thread diameter 222 and a first longitudinal thread spacing 224. The first screw threads 218 are adapted to thread into a first internally threaded tunnel in bone 226. The distal fixation member 202 has second screw threads 208 having a second major thread diameter 210, a second minor thread diameter 212 and a second longitudinal thread spacing 214. The second screw threads 208 are adapted to thread into a second internally threaded tunnel in bone 216.

The second major thread diameter 210 is less than the first minor thread diameter 222. That is, the distal fixation device 202 is adapted to pass longitudinally and substantially without mechanical interference through the first internally threaded tunnel. Thus, the implant 200 can be passed distally directly through the first internally threaded tunnel 226 to the second internally threaded tunnel 216, and threaded substantially simultaneously into the second 216 and the first internally threaded tunnel 226. The first thread spacing 224 is substantially equal to the second thread spacing 214, so that the distal 202 and the proximal fixation device 204 can be threaded into their respective threaded tunnels at the same axial rate with rotation of a single insertion tool engaged through an axial bore 228 through the proximal fixation member 204 and into the distal fixation member 202.

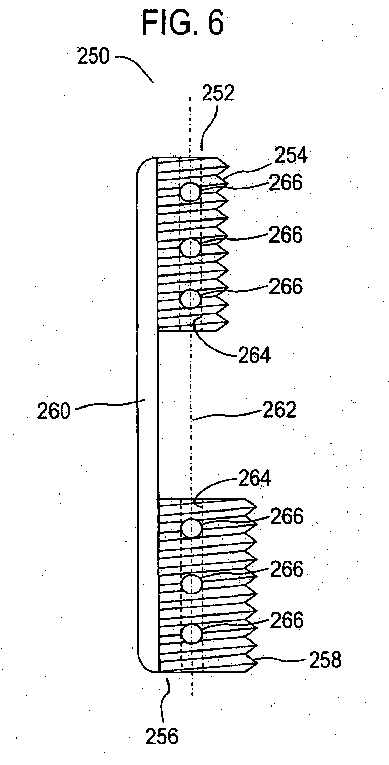

FIG. 6 illustrates another embodiment of a threaded implant 250 of the present invention. The implant 250 includes a proximal fixation member 256 having a first external surface 258, and a distal fixation member 252 having a second external surface 254. A flexible ligament member 260 extends between and interconnects the distal 252 and the proximal fixation member 256. The implant 200 also includes an axis 262, and an axial bore 264 through the proximal fixation member 256 and into the distal fixation member 252. In an embodiment, the axial bore 264 extends distally completely through the distal fixation member 252. One or both of the distal 252 and the proximal fixation member 254 includes one or more transverse bore 266 providing fluid communication between the axial bore 264 and one or both of the first 258 and the second external surface 254, for delivering a fluid to a surgical site. The fluid may be an adhesive, a cement or filler material, a lubricant, a medicant such as pain medication or a healing stimulant, or another fluid. The fluid can be delivered by injection through a cannulated insertion tool positioned in the axial bore 264 and having transverse apertures aligned with the one or more transverse bore 266. In one embodiment, the fluid is an adhesive that enhances the fixation of a fixation member in a bone tunnel.

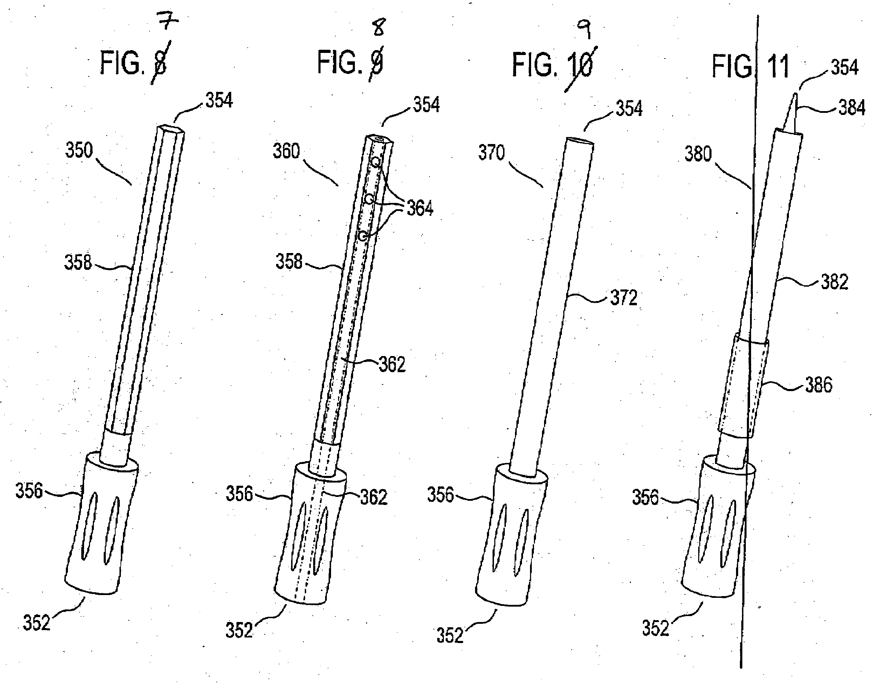

FIG. 7 through FIG. 9 illustrate insertion tools for embodiments of the implants illustrated in FIG. 1 through FIG. 6. FIG. 7 illustrates a hexagonal cross section insertion tool 350 for the implant 100 of FIG. 1 and FIG. 2. The insertion tool 350 has a proximal end 352 and a distal end 354. The insertion tool 350 includes a proximal screwdriver-like handle 356 and a distal, elongated hexagonal external cross section shaft 358 adapted for sliding, removable engagement with the hexagonal internal cross section bore 132 illustrated in the proximal end view 130 of an embodiment of the implant 100. The shaft 358 is fixedly attached to the handle 356. The tool 350 is engaged with the implant 100 by passing the shaft 358 through the axial bore 128 through the proximal fixation member 112 and into the distal fixation member 108. With the tool 350 engaged in the axial bore 128 in both the proximal 112 and the distal fixation member 108, turning the handle 356 rotates both fixation members about the axis 106 simultaneously, for threading the implant 100 into a bone tunnel.

When the distal 108 and proximal fixation member 112 is fully threaded into its respective first 110 and second tunnel 114, as illustrated in FIG. 1, the tool 350 can be fully withdrawn from the bore 128, leaving the implant 100 fixed in place. Alternatively, the tool 350 can be partially withdrawn from the bore 128 to disengage the shaft 358 from the distal fixation member 108 while remaining engaged with the proximal fixation member 112. Rotating the handle 356 with the tool 350 in this partially withdrawn position adjusts tension on the flexible ligament member 116 by rotating the externally threaded proximal fixation member 112 in the internally threaded second tunnel 114, while leaving the distal fixation member 108 stationary. When the tension adjustment is complete, the tool 350 can be fully withdrawn from the implant, leaving the tensioned implant 100 in place.

A cannulated insertion tool can be used to deliver a fluid to an implant of the present invention and to an associated surgical site. FIG. 8 illustrates a cannulated insertion tool 360 constructed in a similar manner to the hexagonal cross section insertion tool 350 illustrated in FIG. 7, with the addition of a cannulation 362 through the handle 356 and the shaft 358. The cannulation 362 is open at the proximal end 352 and in various embodiments is open or closed at the distal end 354. The cannulated tool 360 also includes one or more transverse aperture 364 through which a fluid introduced into the cannulation 362 from the proximal end 352 can be ejected from the cannulated tool 360. In an embodiment, the one or more transverse aperture 364 is aligned with the one or more transverse bore 266 in the implant 250 illustrated in FIG. 6, for delivery of a fluid to a surgical site. In addition to a fluid, gels or powders may also be delivered thorugh the tool 360.

FIG. 9 illustrates an oval cross section insertion tool 370 that is similar in construction to the hexagonal cross section tool 350 of FIG. 7, except that the oval cross section tool 370 includes an oval external cross section distal shaft 372 adapted to removably engage the oval internal cross section bore 136 illustrated in FIG. 3, in the proximal end view 134 of an embodiment of the implant 100.

Any of the implants and tools of the present invention may be supplied to a surgeon as a kit for performing ACL surgery. Packaging the implant and the tool as a kit provides additional convenience for the surgeon and stable. In a kit, the implant may be pre-mounted on the tool to provide even greater convenience for the surgeon and a means for stably and protectively packaging the implant for shipment and storage.

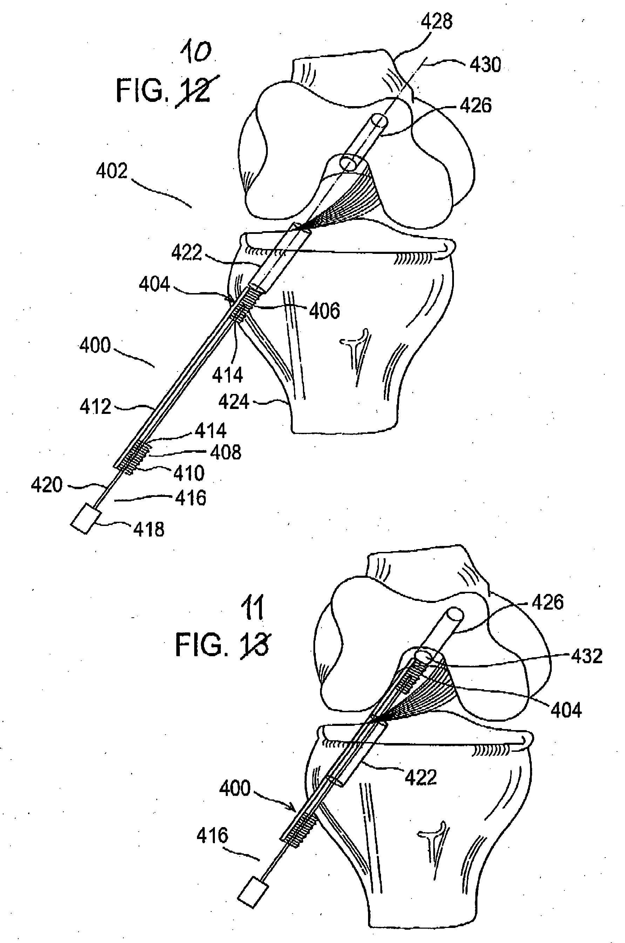

Referring now to FIGS. 10-13, the use of threaded implants and associated installation tools of the present invention in a surgical procedure is illustrated. Implants of the present invention may be used in the surgical repair of any articulated joint in a body, and have particular application to ACL repair surgeries in the human knee. Referring first to FIG. 10, prior to the installation of a threaded implant 400 of the present invention in a knee 402, the knee 402 is prepared and positioned for ACL replacement surgery using conventional surgical tools and conventional surgical procedures. The implant 400 includes an externally threaded proximal fixation member 408 having first external screw threads 410, an externally threaded distal fixation member 404 having second external screw threads 406, and a flexible ligament member 412 extending between and interconnecting the distal fixation member 404 and the proximal fixation member 408. The implant 400 also is seen to have an axial bore 414 for receiving an insertion tool 416. The insertion tool 416 includes a proximal handle 418 and a distal shaft 420 for engagement with the axial bore 414. The implant 400 is mounted on the tool 416 by passing the shaft 420 distally through the axial bore 414 in the proximal fixation member 408 and into the axial bore 414 in the distal fixation member 404. The handle 418 can be rotated to rotate the tool 416 and the implant 400 mounted thereon.

A tibial bone tunnel 422 through a tibia 424 and a femoral bone tunnel 426 in a femur 428 are prepared using conventional surgical tools and techniques. The tibial tunnel 422 and the femoral tunnel 426 share a common axis 430. The tibial tunnel 422 is prepared with an internal diameter and internal screw threads adapted to engage with the first screw threads 410 on the proximal fixation member 408. The femoral tunnel 426 is prepared with an internal diameter and internal screw threads adapted to engage with the second screw threads 406 on the distal fixation member 404. The internal screw threads of the tibial 422 and the femoral tunnel 426 are formed using conventional thread forming techniques and tools. In an embodiment, the internal screw threads in each of the tibial 422 and femoral tunnel 426 are prepared using a threading tap. The implant 400 includes distal and proximal fixation members having unequal diameters, as described herein in association with embodiments of the implant 200 illustrated in FIG. 5. The tibial 422 and the femoral tunnel 426 are prepared with corresponding internal threads adapted to engage the respective proximal 408 and distal fixation member 404.

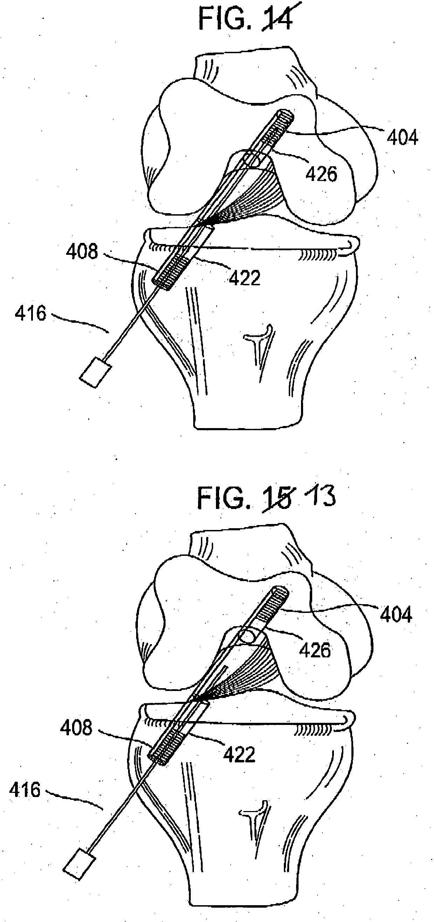

In FIG. 10, the implant 400 is illustrated mounted to the insertion tool 416 and partially threaded into the tibial tunnel 422. The implant 400 is rotated about the axis 430 using the tool 416 to thread the distal fixation device 404 through the tibial tunnel 422, after which the implant is pushed distally to an entrance 432 of the femoral tunnel 426, as illustrated in FIG. 13. The implant 400 includes distal and proximal fixation members having unequal diameters, as described herein in association with embodiments of the implant 200 illustrated in FIG. 5, and the distal fixation member 404 has a major thread diameter that is smaller than a minimum inner diameter of the threaded tibial tunnel 422. The distal fixation member 404 is passed distally through the tibial tunnel 422 and to the entrance 432 of the femoral tunnel 426 without engaging the internal threads of the tibial tunnel 422. and without requiring rotation of the tool 416 and of the implant 400

The distal fixation member 404 is then threaded into the femoral tunnel 426 simultaneously with the proximal fixation member 408 being threaded into the tibial tunnel 422, as illustrated in FIG. 12. In an embodiment, the distal fixation member 404 includes one or more transverse bore, as described in association with the implant 250 illustrated in FIG. 6, and the insertion tool 416 includes one or more corresponding transverse aperture and a cannulation, as described herein in association with embodiments of the cannulated insertion tool 360 illustrated in FIG. 8. In this embodiment, a fluid may be injected into the surgical site through the tool 416 to provide medication or to enhance the fixation of the distal fixation member 404 in the femoral tunnel 426.

Referring now to FIG. 13, the tool 416 has been withdrawn proximally from the distal fixation member 404 positioned in the femoral tunnel 426, while remaining engaged with the proximal fixation member 408 in the tibial tunnel 422. In this position, rotation of the tool 416 rotates the proximal fixation member 408 within the tibial tunnel 422 to adjust tension on the flexible ligament portion 412 of the implant 400. In an embodiment, the proximal fixation member includes one or more transverse bore, as described herein in association with embodiments of the implant 250 illustrated in FIG. 6, and the insertion tool 416 includes one or more corresponding transverse aperture and a cannulation, as described herein in association with embodiments of the cannulated insertion tool 360 illustrated in FIG. 8. In this embodiment, a fluid may be injected into the surgical site through the tool 416 to provide medication or enhance the fixation of the proximal fixation member 408 in the tibial tunnel 422. The tool 416 is fully withdrawn proximally from the implant 400 to complete the installation of the implant 400.

The implants, of the present invention have many advantages. The advantages include providing the surgeon with prepared, pre-sized implants for immediate placement in specified bone tunnels, saving the surgeon time and reducing the surgical skill required to perform an ACL repair surgery relative to conventional bone-tendon bone ACL repair surgeries where the surgeon often is required to manually shape oversized bone blocks to fit bone tunnels. Another advantage of the present invention is that the implant is self-fixating at one or both of the femoral and the tibial end, further reducing the labor and time required of the surgeon in fixating the implant. Further, the self-fixating implants reduce the number of foreign bodies left in the patient after surgery. By providing fixation members fabricated from the material of the bone blocks or their synthetic equivalents, the present invention provides not only fixation members that are integrated with an implant, but fixation members that are unitary with the material of the implant, making the implant a single part. Yet another advantage of the present invention is that it provides an implant that can be positioned in a joint and tensioned with a single insertion tool. Still another advantage of the present invention is that it provides a combination of an implant and a tool that can be used to conveniently inject a medicant or an adhesive selectively into a surgical site to enhance healing or fixation of the implant.

Although this invention has been shown and described with respect to detailed embodiments thereof, it will be understood by those skilled in the art that various changes in form and detail may be made without departing from the scope of the claimed invention.

## Claims

1. An implant (200) for replacing an anterior cruciate ligament in a knee, the implant comprising:
a) a first fixation member (204) having an axis, a first substantially cylindrical external surface about the axis, the first surface defining first external screw threads (218) adapted for threaded engagement with first internal screw threads formed in a tunnel (226) in a tibia adjacent to the knee, the first external screw threads having a first major thread diameter (220) and a first minor thread diameter (222);
b) a second fixation member (202) having a second substantially cylindrical external surface about the axis, the second surface defining second external screw threads (208) adapted for threaded engagement with second internal screw threads formed in a tunnel (216) in a femur adjacent to the knee, the second external screw threads having a second major thread diameter (210) and a second minor thread diameter (212); and
c) a flexible graft ligament member (206) interconnecting the first fixation member (204) and the second fixation member (202);
wherein said major thread diameters of said external screw threads is the outer diameter of said external screw thread measured at peaks of the threads; and said minor thread diameters of said external screw threads is the diameter of said external screw thread measured from the bottom of valleys between the threads;
**characterized in that** the second major thread diameter (210) is smaller than the first minor thread diameter (222).

2. The implant of claim 1 wherein the first fixation member (204), the second fixation member (202) and the ligament member (206) comprise an allograft.

3. The implant of claim 1 wherein the first fixation member (204), the second fixation member (202) and the ligament member (206) comprise a xenograft.

4. The implant of claim 1 wherein at least one of the first (204) and the second (202) fixation member is reinforced with a biocompatible material.

5. The implant of claim 1 wherein the first fixation member (204), the second fixation member (202) and the ligament member (206) comprise one or more synthetic biocompatible material.

6. The implant of claim 1 wherein the first (204) and the second (202) fixation member define an axial bore (228) along the axis, the axial bore extending completely through the first fixation member (204) and at least partially through the second fixation member (202), the axial bore having an internal cross section selected from the group consisting of a polygon and an oval.

7. The implant of claim 6 wherein at least one of the first (204) and the second (202) fixation member further defines a transverse bore (266) in fluid communication with the axial bore (228) and with the respective at least one of the first and the second external surface.

8. A kit for replacing an anterior cruciate ligament in a knee, the kit comprising:
a) an implant according to claim 1; the first fixation member (204) defining an axial bore (228) therethrough; and
b) an implant insertion tool (350) having;
i) an elongated shaft (358) adapted for sliding engagement with the axial bore (228) through the first fixation member (204) and at least partially through the second fixation member (202); and
ii) a handle (356) fixed to the elongated shaft (358) for positioning outside the axial bore.

9. The kit of claim 8 wherein the insertion tool (350) is at least partially cannulated.

10. The kit of claim 8 wherein the implant (200) is pre-mounted to the tool (350).

## Patentansprüche

1. Implantat (200) zum Ersetzen eines vorderen Kreuzbands in einem Knie, wobei das Implantat das Folgende umfasst:
a) ein erstes Fixierungselement (204) mit einer Achse, eine erste, im Wesentlichen zylinderförmige Außenfläche um die Achse, wobei die erste Fläche erste Außengewinde (218) definiert, die für Gewindeeingriff mit ersten, in einem Tunnel (226) in einer Tibia neben dem Knie geformten Innengewinden ausgelegt sind, wobei die ersten Außengewinde einen ersten Hauptgewindedurchmesser (220) und einen ersten Kerngewindedurchmesser (222) aufweisen;
b) ein zweites Fixierungselement (202) mit einer zweiten, im Wesentlichen zylinderförmigen Außenfläche um die Achse, wobei die zweite Fläche zweite Außengewinde (208) definiert, die für Gewindeeingriff mit zweiten, in einem Tunnel (216) in einem Femur neben dem Knie geformten Innengewinden ausgelegt sind, wobei die zweiten Außengewinde einen zweiten Hauptgewindedurchmesser (210) und einen zweiten Kerngewindedurchmesser (212) aufweisen;
c) ein flexibles Bandtransplantatelement (206), welches das erste Fixierungselement (204) und das zweite Fixierungselement (202) miteinander verbindet; wobei die Hauptgewindedurchmesser der Außengewinde der Außendurchmesser des Außengewindes, gemessen an den Spitzen der Gewinde, ist; und wobei die Kerngewindedurchmesser der Außengewinde der Durchmesser des Außengewindes, gemessen vom Boden von Mulden zwischen den Gewinden, ist;
**dadurch gekennzeichnet, dass** der zweite Hauptgewindedurchmesser (210) kleiner als der erste Kerngewindedurchmesser (222) ist.

2. Implantat nach Anspruch 1, wobei das erste Fixierungselement (204), das zweite Fixierungselement (202) und das Bandelement (206) ein Allotransplantat umfassen.

3. Implantat nach Anspruch 1, wobei das erste Fixierungselement (204), das zweite Fixierungselement (202) und das Bandelement (206) ein Xenotransplantat umfassen.

4. Implantat nach Anspruch 1, wobei wenigstens das erste (204) und/oder das zweite (202) Fixierungselement mit einem biokompatiblen Material verstärkt ist.

5. Implantat nach Anspruch 1, wobei das erste Fixierungselement (204), das zweite Fixierungselement (202) und das Bandelement (206) ein oder mehrere synthetische biokompatible Materialien umfassen.

6. Implantat nach Anspruch 1, wobei das erste (204) und das zweite (202) Fixierungselement eine axiale Bohrung (228) entlang der Achse definieren, wobei sich die axiale Bohrung vollständig durch das erste Fixierungselement (204) und wenigstens teilweise durch das zweite Fixierungselement (202) erstreckt, wobei die axiale Bohrung einen inneren Querschnitt aufweist, der aus der aus einem Vieleck und einem Oval bestehenden Gruppe ausgewählt ist.

7. Implantat nach Anspruch 6, wobei wenigstens das erste (204) und/oder das zweite (202) Fixierungselement ferner eine Querbohrung (266) definiert, die mit der axialen Bohrung (228) und mit der jeweiligen wenigstens ersten und/oder zweiten Außenfläche in Fluidverbindung steht.

8. Kit zum Ersetzen eines vorderen Kreuzbands in einem Knie, wobei das Kit das Folgende umfasst:
a) ein Implantat nach Anspruch 1; wobei das erste Fixierungselement (204) eine axiale Bohrung (228) dort hindurch definiert; und
b) ein Implantateinführungswerkzeug (350) mit:
i) einem langgestreckten Schaft (358), der für Gleiteingriff mit der axialen Bohrung (228) durch das erste Fixierungselement (204) und wenigstens teilweise durch das zweite Fixierungselement (202) ausgelegt ist; und
ii) einem Griff (356), der zur Positionierung außerhalb der axialen Bohrung am langgestreckten Schaft (358) fixiert ist.

9. Kit nach Anspruch 8, wobei das Einführungswerkzeug (350) wenigstens teilweise kanüliert ist.

10. Kit nach Anspruch 8, wobei das Implantat (200) am Werkzeug (350) vormontiert ist.

## Revendications

1. Implant (200) pour remplacer un ligament croisé antérieur dans un genou, l'implant comprenant:
a) un premier élément de fixation (204) présentant un axe, une première surface externe sensiblement cylindrique autour de l'axe, la première surface définissant des premiers filets de vis externes (218) aptes à réaliser un engagement vissé avec des premiers filets de vis internes formés dans un tunnel (226) dans un tibia adjacent au genou, les premiers filets de vis externes présentant un premier diamètre de filet majeur (220) et un premier diamètre de filet mineur (222);
b) un second élément de fixation (202) présentant une seconde surface externe sensiblement cylindrique autour de l'axe, la seconde surface définissant des seconds filets de vis externes (208) aptes à réaliser un engagement vissé avec des seconds filets de vis internes formés dans un tunnel (216) dans un fémur adjacent au genou, les seconds filets de vis externes présentant un second diamètre de filet majeur (210) et un second diamètre de filet mineur (212); et
c) un élément de ligament greffé flexible (206) qui interconnecte le premier élément de fixation (204) et le second élément de fixation (202) ;
dans lequel lesdits diamètres de filet majeurs desdits filets de vis externes correspond au diamètre extérieur dudit filet de vis externe mesuré aux crêtes de filets; et lesdits diamètres de filet mineurs desdits filets de vis externes correspond au diamètres dudit filet de vis externe mesuré à partir du fond des vallées entre les filets ;
**caractérisé en ce que** le second diamètre de filet majeur (210) est plus petit que le premier diamètre de filet mineur (222).

2. Implant selon la revendication 1, dans lequel le premier élément de fixation (204), le second élément de fixation (202) et l'élément de ligament (206) comprennent une allogreffe.

3. Implant selon la revendication 1, dans lequel le premier élément de fixation (204), le second élément de fixation (202) et l'élément de ligament (206) comprennent une xénogreffe.

4. Implant selon la revendication 1, dans lequel au moins un parmi le premier (204) et le second (202) éléments de fixation est renforcé avec un matériau biocompatible.

5. Implant selon la revendication 1, dans lequel le premier élément de fixation (204), le second élément de fixation (202) et l'élément de ligament (206) comprennent un ou plusieurs matériau(x) biocompatible(s) synthétique(s).

6. Implant selon la revendication 1, dans lequel les premier (204) et second (202) éléments de fixation définissent un alésage axial (228) le long d'un axe, l'alésage axial s'étendant complètement à travers le premier élément de fixation (204) et au moins partiellement à travers le second élément de fixation (202), l'alésage axial présentant une section transversale interne sélectionnée dans le groupe comprenant les formes polygonale et ovale.

7. Implant selon la revendication 6, dans lequel au moins un des premier (204) et second (202) éléments de fixation définit en outre un alésage transversal (266) en communication fluidique avec l'alésage axial (228) et avec ladite au moins une respective des première et seconde surfaces externes.

8. Kit pour remplacer un ligament croisé antérieur dans un genou, le kit comprenant:
a) un implant selon la revendication 1 ; le premier élément de fixation (204) définissant un alésage axial (228) à travers celui-ci; et
b) un outil d'insertion d'implant (350), comprenant:
i) un arbre allongé (358) apte à réaliser un engagement coulissant avec l'alésage axial (228) à travers le premier élément de fixation (204) et au moins partiellement à travers le second élément de fixation (202); et
ii) une poignée (356) fixée à l'arbre allongé (358) à positionner à l'extérieur de l'alésage axial.

9. Implant selon la revendication 8, dans lequel l'outil d'insertion (350) est au moins partiellement tubulaire.

10. Kit selon la revendication 8, dans lequel l'implant (200) est pré-monté sur l'outil (350).
